(19) Europäisches Patentamt
European Patent Office
Office européen des brevets

(11) Veröffentlichungsnummer : **0 331 930 B1**

(12) # EUROPÄISCHE PATENTSCHRIFT

(45) Veröffentlichungstag der Patentschrift : **03.06.92 Patentblatt 92/23**

(51) Int. Cl.$^5$ : **A61K 7/11**

(21) Anmeldenummer : **89102435.8**

(22) Anmeldetag : **13.02.89**

(54) **Mittel zur Festigung der Frisur und Pflege des Haares.**

Die Akte enthält technische Angaben, die nach dem Eingang der Anmeldung eingereicht wurden und die nicht in dieserPatentschrift enthalten sind.

(30) Priorität : **10.03.88 DE 3807915**

(43) Veröffentlichungstag der Anmeldung : **13.09.89 Patentblatt 89/37**

(45) Bekanntmachung des Hinweises auf die Patenterteilung : **03.06.92 Patentblatt 92/23**

(84) Benannte Vertragsstaaten : **AT BE CH DE ES FR GB GR IT LI NL SE**

(56) Entgegenhaltungen :
**EP-A- 0 155 400**
**SEIFEN-OLE-FETTE-WACHSE, Band 110, Nr. 17, Oktober 1984, Seiten 515-516, Augsburg, DE; K. SEIB et al.: "Neue polyquaternärePolymere mit abgestufter Kationaktivität"**

(73) Patentinhaber : **Wella Aktiengesellschaft**
**Berliner Allee 65**
**W-6100 Darmstadt (DE)**

(72) Erfinder : **Gross, Paul**
**Forstweg 54**
**W-6100 Darmstadt (DE)**
Erfinder : **Henze, Hildegard**
**Georgenstrasse 11**
**W-6100 Darmstadt (DE)**
Erfinder : **Lang, Günther, Dr.**
**Auf der Roten Erde 10 B**
**W-6107 Reinheim 5 (DE)**
Erfinder : **Wendel, Harald**
**Granbengasse 3**
**W-6105 Ober-Ramstadt (DE)**
Erfinder : **Die andere Erfinder haben auf ihre Nennung verzichtet**

EP 0 331 930 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Gegenstand der Erfindung ist ein Mittel zur Festigung der Frisur und Pflege des Haares, welches Tetraoxyethylenlaurylether und ein kationisches Copolymerisat aus Vinylimidazoliummethochlorid und Vinylpyrrolidon enthält.

Mittel zur Festigung der Frisur und Pflege des Haares bestehen üblicherweise aus Lösungen von filmbildenden natürlichen oder synthetischen Polymeren. Als natürliche Polymere kommen beispielsweise Schellack, Alginate, Gelatine, Pektine, Chitosansalze und Cellulosederivate in Betracht. Von den synthetischen Polymeren finden zum Beispiel Polyvinylpyrrolidon, Polyvinylacetat, Polyacrylverbindungen wie Acrylsäure- oder Methacrylsäurepolymerisate, basische Polymerisate von Estern aus diesen beiden Säuren mit Aminoalkoholen beziehungsweise die Salze oder Quaternisierungsprodukte dieser basischen Polymerisate, Polyacrylnitril sowie Copolymerisate aus derartigen Verbindungen, beispielsweise Polyvinylpyrrolidon-Vinylacetat oder Polyvinylpyrrolidon-Dimethylaminoethylmethacrylat, Verwendung.

Weiterhin enthalten Mittel zur Pflege des Haares und Festigung der Frisur zwecks Verbesserung der Naßkämmbarkeit und de Griffes, insbesondere des geschädigten Haares, häufig monomere quaternäre Ammoniumverbindungen wie zum Beispiel Alkyltrimethylammoniumchloride, Dialkyldimethylammoniumchloride, Alkyldimethylbenzylammoniumchloride und Alkylpyridiniumchloride.

Derartige Zusätze quaternärer Ammoniumverbindungen verschlechtern jedoch die physiologische Verträglichkeit solcher Präparate, insbesondere die Augenverträglichkeit.

Aus der eigenen DE-OS 34 01 037 ist ein kosmetisches Mittel zur Pflege und Festigung der Haare auf der Basis einer Kombination aus Tetraoxyethylenlaurylether und einem quaternisierten Copolymerisat aus Vinylpyrrolidon und Dimethylaminoethylmethacrylat bekannt, bei dem ein Zusatz derartiger quaternärer Ammoniumverbindungen nicht erforderlich sein soll.

Die haarkonditionierenden Eigenschaften dieser Mittel sind jedoch, insbesondere bei geschädigtem Haar, nicht völlig befriedigend (siehe Testbeispiel I). Weiterhin ist dieses Mittel aufgrund des hohen Preises des verwendeten quaternisierten Copolymerisates aus Vinylpyrrolidon und Dimethylaminoethylmethacrylat verhältnismäßig teuer.

Demgegenüber wurde nun gefunden, daß eine gleichzeitige hervorragende Konditionierung und gute Festigung der Haare durch die Verwendung einer synergistischen Kombination von Tetraoxyethylenlaurylether und einem kationischen Copolymerisat von Vinylimidazoliummethochlorid und Vinylpyrrolidon erzielt werden kann.

Gegenstand der vorliegenden Erfindung ist daher ein Mittel zur Festigung der Frisur und Pflege des Haares auf der Basis einer wäßrigen, alkoholischen oder wäßrig-alkoholischen Lösung eines quaternisierten Copolymerisates des Vinylpyrrolidons und eines Tetraoxyethylenlaurylethers sowie gegebenenfalls einem Treibmittel und üblichen Zusätzen, welches dadurch gekennzeichnet ist, daß es

(a) als quaternisiertes Copolymerisat des Vinylpyrrolidons 0,1 bis 10,0 Gewichtsprozent eines kationischen Copolymerisates aus Vinylimidazoliummethochlorid und Vinylpyrrolidon und

(b) 0,1 bis 5,0 Gewichtsprozent Tetraoxyethylenlauryl ether

enthält.

Als kationisches Copolymerisat aus Vinylimidazoliummethochlorid und Vinylpyrrolidon sind besonders die Handelsprodukte Luviquat® FC 370, Luviquat® FC 550 und Luviquat® FC 905 der Firma BASF AG, Ludwigshafen (Bundesrepublik Deutschland) geeignet. Diese Copolymerisate (CTFA Bezeichnung: Polyquaternium--16) besitzendie folgende Strukturformel (I):

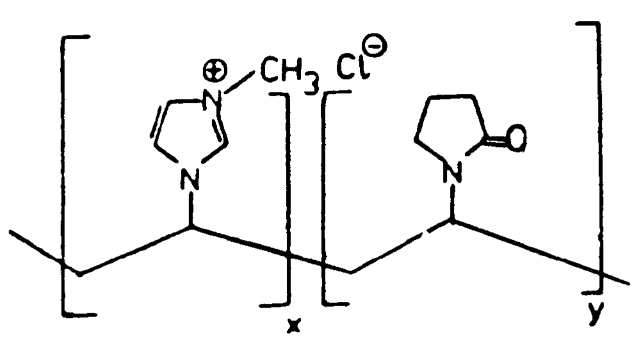

(I),

mit

x = 0,30 - 0,95

y = 0,05 - 0,70
x + y = 1.

Das mittlere Molekulargewicht des kationischen Copolymerisates der Formel (I) liegt vorzugsweise zwischen 40.000 und 1.000.000 .

Von den genannten Copolymerisaten ist ein Copolymerisat aus 95 Molprozent Vinylimidazoliummethochlorid und 5 Molprozent Vinylpyrrolidon (Hamdelsmame: Luviquat® FC 905 der Firma BASF) mit einem mittleren Molekulargewicht von etwa 80.000 besonders bevorzugt.

Der Tetraoxyethylenlaurylether der Formel (II)

$$CH_3\,(CH_2)_{10}CH_2\,(OCH_2CH_2)_4\,OH \qquad (II)$$

ist ein mit 4 Mol Ethylenoxid oxethylierter Laurylalkohol.

In dem erfindungsgemäßen Mittel soll der Tetraoxyethylenlaurylether in einer Menge von 0,1 bis 5 Gewichtsprozent, vorzugsweise in einer Menge von 0,5 bis 2 Gewichtsprozent, enthalten sein.

Der Gehalt an dem kationischen Copolymerisat der Komponente (a) beträgt 0,1 bis 10 Gewichtsprozent, vorzugsweise 1 bis 3,5 Gewichtsprozent.

Das erfindungsgemäße Mittel weist vorzugsweise einen pH-Wert zwischen 2 und 11 auf und kann in Form einer wäßrigen, alkoholischen oder wäßrig-alkoholischen Lösung vorliegen.

Als Alkohol kommen hierbei insbesondere die für kosmetische Zwecke üblicherweise verwendeten niederen Alkohole mit 1 bis 4 Kohlenstoffatomen, wie zum Beispiel Ethanol und Isopropanol, in Betracht. Diese Alkohole können in dem erfindungsgemäßen Mittel in einer Menge von 0 bis 99,8 Gewichtsprozent enthalten sein.

Selbstverständlich können auch übliche haarkosmetische Zusätze wie beispielsweise Parfümöle, Kräuterextrakte, bakterizide oder fungizide Stoffe, Antischuppenmittel, Lösungsvermittler für Parfümöle, natürliche oder synthetische Harze, wie zum Beispiel Chitosanderivate, Polyvinylpyrrolidon und Polyvinylpyrrolidon-Vinylacetat-Copolymere, in dem erfindungsgemäßen Mittel enthalten sein, soweit solche Zusätze nützlich und zweckmäßig erscheinen. Weiterhin können auch Anfärbefarbstoffe zur Anfärbung des Präparates oder direkt auf das Haar aufziehende Farbstoffe zur gleichzeitigen Tönung des Haares enthalten sein.

Von letzteren Farbstoffen, die einzeln oder in Mischung vorliegen können, seien beispielsweise die folgenden Klassen erwähnt: Aromatische Nitrofarbstoffe (zum Beispiel 1,4-Diamino-2-nitro-benzol, Pikraminsäure, 1-Hydroxy-2-amino-4-nitro-benzol und 1,4-Bis(2-hydroxyethyl)-amino-2-nitro-5-chlor-benzol), Azofarbstoffe (zum Beispiel C.I. 14 805 - Acid Brown 4), Anthrachinonfarbstoffe (zum Beispiel C.I. 61 105 -Disperse Violet 4) und Triphenylmethanfarbstoffe (zum Beispiel C.I. 42 535 - Basic Violet 1), wobei die Farbstoffe dieser Klassen je nach Art ihrer Substituenten sauren, nichtionogenen oder basischen Charakter haben können. Ihre Gesamtkonzentration in dem erfindungsgemäßen Mittel beträgt üblicherweise etwa 0,01 bis 2,0 Gewichtsprozent.

Das erfindungsgemäße Mittel kann auch unter Zusatz eines Treibmittels in einem Druckbehälter abgefüllt werden, wobei das Präparat vorzugsweise als Schaum entnommen wird und mittels eines mit einer Auftragdüse versehenen Ventils leicht dosiert und bequem auf dem Haar verteilt werden kann. Als Treibmittel sind beispielsweise leicht flüchtige Fluorchlorkohlenwasserstoffe, wie zum Beispiel Difluordichlormethan oder Trichlormonofluormethan, Tetrafluordichlorethan oder niedere Alkane, wie zum Beispiel n-Butan, i-Butan und Propan oder auch Dimethylether sowie ferner bei den in Betracht kommenden Drücken gasförmig vorliegende Treibmittel, wie beispielsweise $N_2$, $N_2O$ und $CO_2$, sowie Gemische der vorstehend genannten Verbindungen geeignet. Die Treibmittel sind in diesen Mitteln zweckmäßigerweise in einer Menge von etwa 2 bis 10 Gewichtsprozent enthalten.

Das erfindungsgemäße Mittel wird, üblicherweise nach der Haarwäsche, in dem handtuchtrockenen Haar in einer Menge von etwa 5 bis 20 Gramm, je nach Haarfülle, verteilt. Anschließend wird das Haar durchgekämmt und in üblicher Weise entweder unmittelbar gefönt oder zunächst auf Wasserwellwickler gewickelt und sodann getrocknet.

Das Mittel zur Festigung der Frisur und Pflege des Haares gemäß der vorliegenden Erfindung bewirkt bei gleichzeitig guter Festigung der Frisur eine überraschende un ausgezeichnete Verbesserung sowohl der Kämmbarkeit des feuchten Haares als auch von Glanz und Griff des trockenen Haares. Insbesondere wird die Gleitwirkung von Kamm oder Bürste im feuchten Haar nach der Applikation des erfindungsgemäßen Mittels wesentlich verbessert, wodurch das Fönen der Haare oder das Aufwickeln auf Wasserwellwickler wesentlich erleichtert wird. Besonders deutlich kommen die vorstehend genannten Vorteile der erfindungsgemäßen Kombination aus Tetraoxyethylenlaurylether und einem Copolymerisat von Vinylimidazoliummethochlorid und Vinylpyrrolidon bei stark geschädigtem Haar zur Geltung.

Bei dem erfindungsgemäßen Mittel zur Festigung der Frisur und Pflege des Haares kann auf Grund seiner ausgezeichneten kämmbarkeitsverbessernden Wirkung ganz auf die Verwendung quaternärer Ammoniumverbindung verzichtet werden, es sei denn, man setzt sie dem Mittel in geringer Konzentration (etwa bis zu 0,2 Gewichtsprozent) als Konservierungsmittel zur Erzielung bakterizider oder fungizider Eigenschaften zu.

Ein weiterer Vorteil des erfindungsgemäßen Mittels ist sein, im Vergleich zu aus der Literatur - beispielsweise der DE-OS 34 01 037 - bekannten Mitteln, niedriger Preis. So kostet das als Komponente (a) verwendete Copolymerisat aus Vinylimidazoliummethochlorid und Vinylpyrrolidon nur etwa ein Drittel des Preises des in der DE-OS 34 01 037 verwendeten kationischen Copolymerisates.

Die beobachtete ausgezeichnete Verbesserung der Kämmbarkeit des feuchten Haares und die Verbesserung von Glanz und Griff ist überraschend und nur synergistisch erklärbar, da der Tetraoxyethylenlaurylether oder das kationische Copolymerisat aus Vinylimidazoliummethochlorid und Vinylpyrrolidon in entsprechender Lösung allein auf das Haar aufgebracht(siehe Testbeispiele C und D) nur eine mäßige und unbefriedigende Verbesserung der genannten Kriterien bewirkt.

Weiterhin wurde durch die nachstehenden Testbeispiele E, F, G und H nachgewiesen, daß andere Fettalkoholpolyoxyethylenether als Tetraoxyethylenlaurylether die für eine Verwendung in den erfindungsgemäßem Mitteln erforderliche synergistische Wirkung nicht aufweisen.

**Beispiele**

Beispiel 1

Mittel in Form einer wäßrigen Lösung

| | |
|---|---|
| 1,2 g | Tetraoxyethylenlaurylether |
| 2,5 g | kationisches Copolymerisat aus Vinylimidazoliummethochlorid und Vinylpyrrolidon im Verhältnis 95 : 5 |
| 96,3 g | Wasser, vollentsalzt |
| 100,0 g | |

Beispiel 2

Mittel in Form einer wäßrig-alkoholischen Lösung

| | |
|---|---|
| 0,7 g | Tetraoxyethylenlaurylether |
| 2,0 g | kationisches Copolymerisat aus Vinylimidazoliummethochlorid und Vinylpyrrolidon im Verhältnis 95 : 5 |
| 20,0 g | Ethanol |
| 77,3 g | Wasser, vollentsalzt |
| 100,0 g | |

Beispiel 3

Mittel in Form einer Druckgaspackung

| | |
|---|---|
| 1,5 g | Tetraoxyethylenlaurylether |
| 2,5 g | kationisches Copolymerisat aus Vinylimidazoliummethochlorid und Vinylpyrrolidon im Verhältnis 95 : 5 |
| 10,0 g | Isopropanol |
| 86,0 g | Wasser, vollentsalzt |
| 100,0 g | |

Abfüllung:

| | |
|---|---|
| 96,0 g | Flüssigkeit der vorstehenden Zusammensetzung |
| 4,0 g | Propan |

Beispiel 4

Mittel in Form einer Druckgaspackung

| | |
|---|---|
| 1,4 g | Tetraoxyethylenlaurylether |
| 2,0 g | kationisches Copolymerisat aus Vinylimidazoliummethochlorid und Vinylpyrrolidon im Verhältnis 50 : 50 |
| 8,0 g | Ethanol |
| 88,6 g | Wasser, vollentsalzt |
| 100,0 g | |

Abfüllung:

| | |
|---|---|
| 96,0 g | Flüssigkeit der vorstehenden Zusammensetzung |
| 2,0 g | Propan |
| 1,6 g | Butan |
| 0.4 g | Dimethylether |

Beispiel 5

Mittel in Form einer wäßrig-alkoholischen Lösung

| | |
|---|---|
| 1,20 g | Tetraoxyethylenlaurylether |
| 2,50 g | kationisches Copolymerisat aus Vinylimidazoliummethochlorid und Vinylpyrrolidon im Verhältnis 30 : 70 |
| 0,50 g | Polyvinylpyrrolidon |
| 0,05 g | Basic Violet (C. I. Nr. 42 535) |
| 5,00 g | Ethanol |
| 90,75 g | Wasser, vollentsalzt |
| 100,00 g | |

Alle in der vorliegenden Anmeldung angegebenen Prozentzahlen stellen Gewichtsprozente dar.

## Versuchsbeispiele

### Testbeispiel A

Nach einer vorangegangenen Haarwäsche wurde das handtuchtrockene Haar von 20 Versuchspersonen mit einem Mittel gemäß Beispiel 1 behandelt.

Zehn der Versuchspersonen hatten normales Haar. Die restlichen zehn Personen hatten geschädigtes bis stark geschädigtes Haar. Um eindeutige Ergebnisse zu erhalten und von Versuchsperson zu Versuchsperson abweichende Haarqualitäten auszuschalten, wurde das Haar in der Mitte gescheitelt und auf die eine Hälfte des Haares, in Abhängigkeit von der Haarfülle, 2,4 bis 6 g des Mittels aufgetragen, während die andere Hälfte unbehandelt blieb. Anschließend wurde jede Hälfte des Haares für sich durchgekämmt, auf Wasserwellwickler gewickelt und getrocknet.

Schließlich wurden die Wickler entfernt und das Haar frisiert. So konnte die Wirkung des Präparates von einer friseur-fachlichen Expertengruppe sicher beurteilt werden. Eine Bewertung erfolgte nach dem Schema für die zusammengefaßten Kriterien der Naßkämmbarkeit, Haltbarkeit der Frisur, Lockigkeit, Glanz, Griff und der statischen Aufladung des Haares.

Benotung:

1 = sehr gut

2 = gut

3 = ausreichend

4 = mangelhaft

Das Ergebnis des Versuches für die so behandelte Haarhälfte im Vergleich zur unbehandelten Hälfte zeigt die nachstehende Tabelle 1.

Tabelle 1

| | Note 1 | Note 2 | Note 3 | Note 4 |
|---|---|---|---|---|
| Anzahl der Versuchs-personen | 18 | 1 | 1 | 0 |

Testbeispiel B

Die Haare einer weiteren Gruppe von 18 Versuchspersonen wurden mit einem Mittel gemäß Beispiel 3 in gleicher Weise wie im Testbeispiel A behandelt. Zehn Personen der Gruppe hatten geschädigtes bis stark geschädigtes Haar. Die restlichen 8 Personen hatten normales Haar. Die Ergebnisse dieses Tests sind in der nachstehenden Tabelle 2 zusammengefaßt.

Tabelle 2

| | Note 1 | Note 2 | Note 3 | Note 4 |
|---|---|---|---|---|
| Anzahl der Versuchspersonen | 16 | 2 | 0 | 0 |

Testbeispiel C

Zur Überprüfung des synergistischen Effektes wurden die Haare einer weiteren Testgruppe von 14 Personen, wie im Testbeispiel A beschrieben, halbseitig mit einer Zusammensetzung gemäß Beispiel 1 behandelt, jedoch wurde der Tetraoxyethylenlaurylether durch die entsprechende Menge an Wasser ersetzt. Die andere Hälfte des Haares blieb unbehandelt. 5 Versuchspersonen hatten normales Haar und 9 Personen hatten geschädigtes Haar. Das Versuchsergebnis ist in Tabelle 3 wiedergegeben.

Tabelle 3

| | Note 1 | Note 2 | Note 3 | Note 4 |
|---|---|---|---|---|
| Anzahl der Versuchspersonen | 0 | 2 | 7 | 5 |

Testbeispiel D

Zur weiteren Überprüfung des synergistischen Effektes wurde eine vierte Testgruppe von 13 Personen, von denen sechs Personen normales und 7 Personen stark geschädigtes Haar hatten, in gleicher Weise wie in Testbeispiel A beschrieben, mit einer Zusammensetzung gemäß Beispiel 1 halbseitig behandelt, jedoch enthielt das Mittel kein kationisches Copolymerisat aus Vinylimidazoliummethochlorid und Vinylpyrrolidon. In der Zusammensetzung nach Beispiel 1 wurde dabei das kationaktive Copolymerisat durch den entsprechenden Gewichtsanteil an Wasser ersetzt. Die zweite Hälfte des Haares blieb unbehandelt. Das Ergebnis des Versuches ist in der nachstehenden Tabelle 4 wiedergegeben.

Tabelle 4

| | Note 1 | Note 2 | Note 3 | Note 4 |
|---|---|---|---|---|
| Anzahl der Versuchs-personen | 0 | 2 | 6 | 5 |

Ein Vergleich der Tabellen 1 und 2 sowohl mit der Tabelle 3 als auch mit der Tabelle 4 zeigt deutlich, daß die in den erfindungsgemäßen Mitteln enthaltene Kombination eine synergistische Wirkung im Vergleich zur Wirkung der Einzelkomponenten besitzt.

Testbeispiel E

Die Haare einer Testgruppe von 10 Personen mit geschädigtem bis stark geschädigtem Haar wurden analog Testbeispiel A halbseitig mit einer Präparatezusammensetzung nach Beispiel 1 behandelt, in dem jedoch der Tetraoxyethylenlaurylether mengengleich durch Trioxyethylenstearylether ersetzt worden war. Die andere Seite des Haares blieb unbehandelt. Das Versuchergebnis zeigt die nachstehende Tabelle 5.

Tabelle 5

| | Note 1 | Note 2 | Note 3 | Note 4 |
|---|---|---|---|---|
| Anzahl der Versuchs-personen | 0 | 2 | 5 | 3 |

Testbeispiel F

Es wurden die Haare einer Gruppe von 10 Versuchspersonen mit geschädigtem Haar bis starke geschädigtem Haar analog Testbeispiel A halbseitig mit einer Präparatezusammensetzung nach Beispiel 1 behandelt, in dem jedoch der Tetraoxyethylenlaurylether mengengleich durch Decaoxyethylenisostearylether ersetzt worden war. Die andere Seite des Haares blieb unbehandelt. Das Ergebnis zeigt die nachstehende Tabelle 6.

Tabelle 6

| | Note 1 | Note 2 | Note 3 | Note 4 |
|---|---|---|---|---|
| Anzahl der Versuchs-personen | 0 | 2 | 6 | 2 |

Die vorstehenden Testbeispiele E und F zeigen, daß unter den Polyoxyethylenlaurylethern nur der Tetraoxyethylenlaurylether einen deutlichen synergistischen Effekt aufweist.

Testbeispiel G

Eine weitere Gruppe von 10 Versuchspersonen mit geschädigtem bis stark geschädigtem Haar wurde analog Testbeispiel A halbseitig mit einer Zusammensetzung gemäß Beispiel 1 behandelt, in dem jedoch der Tetraoxyethylenlaurylether mengengleich durch Trioxyethylenlaurylether ersetzt worden war. Die andere Seite des Haares blieb unbehandelt. Das Ergebnis des Versuches zeigt die nachstehende Tabelle 7.

Tabelle 7

| | Note 1 | Note 2 | Note 3 | Note 4 |
|---|---|---|---|---|
| Anzahl der Versuchs-personen | 0 | 4 | 5 | 1 |

Testbeispiel H

Ebenso wurde eine Testgruppe von 10 Personen mit geschädigtem bis stark geschädigtem Haar analog Testbeispiel A halbseitig mit einer Zusammensetzung gemäß Beispiel 1 behandelt, in dem jedoch der Tetraoxyethylenlaurylether mengengleich durch Pentaoxyethylenlaurylether ersetzt worden war. Die andere Seite des Haares blieb unbehandelt.

Das Ergebnis zeigt die nachstehende Tabelle 8.

Tabelle 8

| | Note 1 | Note 2 | Note 3 | Note 4 |
|---|---|---|---|---|
| Anzahl der Versuchs-personen | 0 | 5 | 5 | 0 |

Die Resultate der vorstehenden Testbeispiele E bis H zeigen, daß andere Fettalkoholpolyoxyethylenether als Tetraoxyethylenlaurylether für eine erfindungsgemäße Verwendung ungeeignet sind.

Die überraschende und ausgezeichnete synergistische Verbesserung der haarkonditionierenden Eigenschaften und die sehr gute Festigung der Frisur, insbesondere bei stark geschädigtem Haar, ist nach den beschriebenen Versuchsergebnissen auf die erfindungsgemäße Kombination von Tetraoxyethylenlaurylether mit einem kationischen Copolymerisat aus Vinylimidazoliummethochlorid und Vinylpyrrolidon beschränkt und ist mit anderen Fettalkoholpolyoxyethylenethern nicht zu erzielen.

Testbeispiel I

Ein Mittel gemäß Beispiel 1 der vorliegenden Erfindung (Mittel I) wurde im Halbseitenversuch mit einem Mittel gemäß Beispiel 1 der DE-OS 34 01 037 (Mittel II) verglichen.

Hierzu wurde das gewaschene, handtuchtrockene Haar von 12 Versuchspersonen, deren Haare mäßig

bis stark geschädigt waren, im Parallelvergleich mit Mittel I und Mittel II behandelt. Um eindeutige Ergebnisse zu erhalten und von Versuchsperson zu Versuchsperson abweichende Haarqualitäten auszuschalten, wurde das Haar in der Mitte gescheitelt und die eine Hälfte des Haares (in Abhängigkeit von der Haarfülle) mit 2,4 bis 6 g des Mittels I behandelt, während die andere Hälfte des Haares mit der gleichen Menge des Mittels II behandelt wurde. Anschließend wurde jede Hälfte des Haares für sich durchgekämmt, auf Wasserwellwickler gewickelt und sodann getrocknet. Anschließend wurden die Wickler entfernt und das Haar frisiert. Die Ergebnisse der friseur-fachlichen Beurteilung sind in der nachfolgenden Tabelle 9 zusammengefaßt.

Tabelle 9

| | Mittel I | Mittel II |
|---|---|---|
| Gleitwirkung von Kamm und Bürste im feuchten Haar | sehr gut | ausreichend |
| Glanz und Griff des trockenen Haares | sehr gut | ausreichend |
| Frisierbarkeit und Halt der Frisur | gut | gut |

Wie der vorstehende Vergleichsversuch I zeigt, ist das erfindungsgemäße Mittel (I) dem aus der DE-OS 34 01 037 bekannten Mittel (II) in Bezug auf seine kämmbarkeitsverbessernden Eigenschaften sowie Glanz und Griff des behandelten Haares eindeutig überlegen.

**Patentansprüche**

1. Mittel zur Festigung der Frisur und Pflege des Haares auf der Basis einer wäßrigen, alkoholischen oder wäßrig-alkoholischen Lösung eines quaternisierten Copolymerisates des Vinylpyrrolidons und eines Tetraoxyethylenlaurylethers sowie gegebenenfalls einem Treibmittel und üblichen Zusätzen, dadurch gekennzeichnet, daß es

(a) als quaternisiertes Copolymerisat des Vinylpyrrolidons 0,1 bis 10,0 Gewichtsprozent eines kationischen Copolymerisates aus Vinylimidazoliummethochlorid und Vinylpyrrolidon und

(b) 0,7 bis 5,0 Gewichtsprozent Tetraoxyethylenlaurylether

enthält.

2. Mittel nach Anspruch 1, dadurch gekennzeichnet, daß es 1 bis 3,5 Gewichtsprozent eines kationischen Copolymerisates aus Vinylimidazoliummethochlorid und Vinylpyrrolidon enthält.

3. Mittel nach Anspruch 7, dadurch gekennzeichnet, daß es 0,5 bis 2,0 Gewichtsprozent Tetraoxyethylenlaurylether enthält.

4. Mittel nach mindestens einem der Ansprüche 1 bis 3, dadurch gekennzeichnet, daß es ein kationisches Copolymerisat aus Vinylimidazoliummethochlorid und Vinylpyrrolidon der Formel (I)

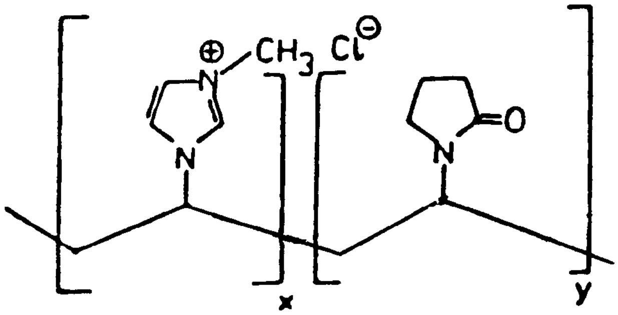

(I),

wobei gilt
x = 0,30 - 0,95
y = 0,05 - 0,70
und
x + y = 1,
und einem Molekulargewicht von 40.000 bis 1.000.000 enthält.

5. Mittel nach mindestens einem der Ansprüche 1 bis 4, dadurch gekennzeichnet, daß es zur gleichzeitigen Tönung des Haares 0,01 bis 2,0 Gewichtsprozent eines direkt auf das Haar aufziehenden Farbstoffes enthält.

6. Mittel nach Anspruch 5, dadurch gekennzeichnet, daß der direkt auf das Haar aufziehende Farbstoff ausgewählt ist aus aromatischen Nitrofarbstoffen, Azofarbstoffen, Anthrachinonfarbstoffen und Triphenylmethanfarbstoffen.

7. Mittel nach mindestens einem der Ansprüche 1 bis 6, dadurch gekennzeichnet, daß es 2 bis 10 Gewichtsprozent eines Treibmittels enthält, daß ausgewählt ist aus n-Butan, i-Butan, Propan, Difluordichlormethan, Trichlormonofluormethan, Tetrafluordichlorethan, Dimethylether, $N_2$, $N_2O$ und $CO_2$ sowie Mischungen dieser Verbindungen.

## Claims

1. A hair care and setting agent based on an aqueous, alcoholic or aqueous-alcoholic solution of a quaternised copolymer of vinyl pyrrolidone and a tetraoxyetlhylene lauryl ether and possibly a propellant and conventional additives, characterised in that

(a) as a quaternised copolymer of vinyl pyrrolidone it contains 0.1 to 10% by weight of a cationic copolymer of vinyl imidazolium methochloride and vinyl pyrrolidone, and

(b) 0.1 to 5% by weight tetraoxyethylene lauryl ether.

2. An agent according to Claim 1, characterised in that it contains 1 to 3.5% by weight of a cationic copolymer of vinyl imidazolium methochloride and vinyl pyrrolidone.

3. An agent according to Claim 1, characterised in that it contains 0.5 to 2.0% by weight tetraoxyethylene lauryl ether

4. An agent according to at least one of Claims 1 to 3, characterised in that it contains a cationic copolymner of vinyl imidazolium methochloride and vinyl pyrrolidone to the formula (I)

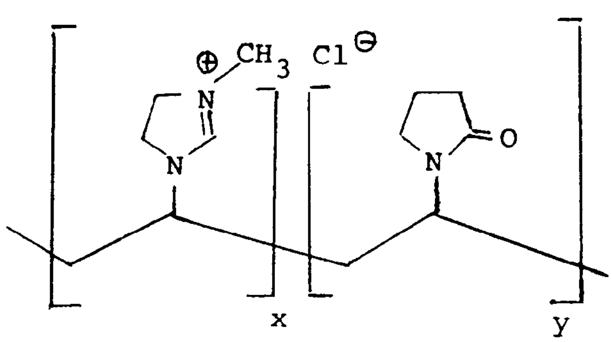

(I)

in which
x = 0.30 - 0.95

y = 0.05 - 0.70
and
x + y = 1,
with a molecular weight of 40,000 to 1,000,000.

5. An agent according to at least one of Claimns 1 to 4, characterised in that for simultaneous tinting of the hair, it contains 0.01 to 2.0% by weight of a colouring agent for direct application to the hair.

6. An agent according to Claim 5, characterised in that the colouring agent for direct application to the hair is selected from aromatic nitrodyes, azodyes, anthraquinone dyes and triphenyl methane dyes.

7. An agent according to at least one of Claims 1 to 6, characterised in that it contains 2 to 10% by weight of a propellant selected from n-butane, i-butane, propane, difluorodichloromethane, trichloromonofluoromethane, tetrafluorodichloroethane, dimethyl ether, $N_2$, $N_2O$ and $CO_2$ as well as mixtures of these compounds.

## Revendications

1. Composition pour fixer la coiffure et le traitement des cheveux, à partir d'une solution aqueuse, alcoolique ou hydroalcoolique d'un copolymérisat quaternisé de vinylpyrrolidone et d'un éther de tétraoxyéthylènelauryle ainsi que le cas échéant d' un agent propulsif et les additifs usuels, caractérisée en ce qu'elle contient:
a) en tant que copolymérisat cationique de vinylpyrrolidone quaternisé de 0,1 à 10% en poids d'un copolymérisat cationique de méthochlorure de vinylimidazolium et de vinylpyrrolidone, et
b) de 0,1 à 5% en poids d'éther de tétraoxyéthylènelauryle.

2. Composition selon la revendication 1, caractérisée en ce qu'il contient de 1 à 3,5% en poids d'un copolymérisat cationique de méthochlorure de vinylimidazolium et de vinylpyrrolidone.

3. Composition selon la revendication 1, caractérisée en ce qu'elle contient de 0,5 à 2,0% en poids d'éther de tétraoxyéthylène-lauryle.

4. Composition selon au moins l'une des revendications 1 à 3, caractérisée en ce qu'elle contient un copolymérisat cationique de méthochlorure de vinylimidazolium et de vinylpyrrolidone de formule:

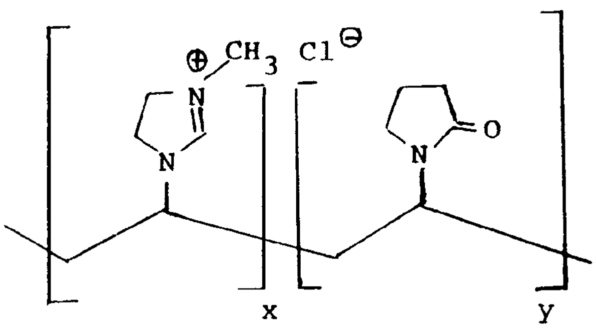

(I)

dans laquelle
x vaut de 0,30 à 0,95
y vaut de 0,05 à 0,70
avec
x + y = 1
le poids moléculaire allant de 40.000 100.000.

5. Composition selon au moins l'une des revendications 1 à 4, caractérisée en ce qu'il contient pour le nuançage simultané des cheveux, de 0,01 à 2% en masse d'un colorant se fixant directement sur les cheveux.

6. Composition selon la revendication 5, caractérisée en ce que le colorant se fixant directement sur les cheveux est choisi parmi les colorants nitrés aromatiques, les colorants azoïques, les colorants anthraquinoniques, et les colorants dérivés du triphénylméthane.

7. Composition selon au moins l'une des revendications 1 à 6, caractérisé qu'il contient de 2 à 10% en poids d'un agent propulsif qui est choisi parmi le N-butane, l'isobutane, le propane, le difluorodichlorométhane, le trichloromonofluorométhane, le tetrafluorodichloroethane, le diméthyléther, $N_2$, $N_2O$ et $CO_2$, ainsi que des mélanges de ces composés.